# EUROPÄISCHE PATENTSCHRIFT

(11) **EP 0 989 121 B1**
(45) Veröffentlichungstag und Bekanntmachung des Hinweises auf die Patenterteilung: **16.11.2005**
(21) Anmeldenummer: 99117319.6
(22) Anmeldetag: 03.09.1999
(51) Int. Cl.: C07D 211/88, A61K 31/4468, A61P 37/02

(54) **Substituierte Benzamide und ihre Verwendung als Immunmodulatoren**
Substituted benzamides and their use as immunomodulators
Benzamides substitués et leur utilisation comme immunomodulateurs

(30) Priorität: 24.09.1998 DE 19843793
(43) Veröffentlichungstag der Anmeldung: 29.03.2000
(73) Patentinhaber: Grünenthal GmbH, 52078 Aachen (DE)
(72) Erfinder: Germann, Tieno, Dr., 52134 Herzogenrath (DE); Frosch, Stefanie, Dr., 52078 Aachen (DE); Zimmer, Oswald, Dr., 52146 Würselen (DE)

(56) Entgegenhaltungen:
- MOLLER D R ET AL: "Inhibition of IL-12 production by thalidomide" J. IMMUNOL., Bd. 159, Nr. 10, 1997, Seiten 5157-5161, XP002125907

## Beschreibung

Die Erfindung betrifft substituierte Benzamide der allgemeinen Formel I 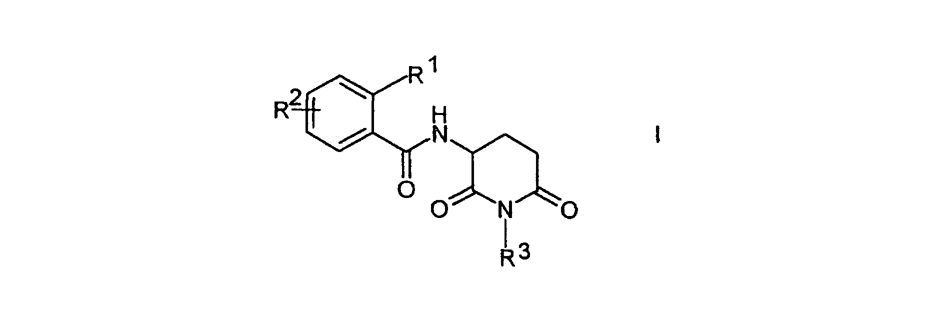 und ihre Verwendung in Arzneimitteln.

Autoimmunerkrankungen entstehen aufgrund einer Reaktivität des Immunsystems gegen körpereigene Strukturen. Dabei ist die normalerweise vorhandene Toleranz gegenüber körpereigenem Gewebe aufgehoben. In der Pathogenese der verschiedenen Autoimmunerkrankungen spielen neben Antikörpern insbesondere T-Lymphozyten und Monozyten/Makrophagen eine entscheidende Rolle. Aktivierte Monozyten/Makrophagen sezernieren eine Vielzahl verschiedener entzündungsfördernder Mediatoren, die direkt oder indirekt für die Zerstörung der von der Autoimmunerkrankung betroffenen Gewebe verantwortlich sind. Die Aktivierung von Monozyten/Makrophagen erfolgt entweder in der Interaktion mit T-Lymphozyten oder über bakterielle Produkte wie Lipopolysaccharid (LPS). Die durch verschiedene bakterielle Produkte induzierte Aktivierung von Monozyten/Makrophagen und Granulozyten ist darüber hinaus charakteristisch für allgemeine Entzündungsreaktionen.

Die Bedeutung des Gleichgewichtes zwischen entzündungsfördernden (z.B. Interleukin IL-12) und entzündungshemmenden Zytokinen (z.B. Interleukin IL-10) für die Entwicklung und den Verlauf von Entzündungen bzw. Autoimmunerkrankungen ist aufgrund zahlreicher tierexperimenteller und erster klinischer Untersuchungen klar dokumentiert. In verschiedenen Tiermodellen für Erkrankungen, wie Rheumatoide Arthritis, Multiple Sklerose, Diabetes mellitus sowie entzündliche Haut- und Schleimhauterkrankungen, zeigt sich die pathophysiologische Bedeutung von IL-12 (Immunol. Today 16/8: 383-387, 1995; J. Immunol. 155: 4661-4668,1995; J. Exp. Med. 182: 1281-1290, 1995; J. Exp. Med. 187/4: 537-546, 1998). Durch Applikation von IL-12 ließ sich die jeweilige Erkrankung auslösen bzw. nach Neutralisierung von endogenem IL-12 zeigte sich ein abgeschwächter Krankheitsverlauf bis hin zu einer Heilung der Tiere.

Bei entzündlichen Darmerkrankungen findet sich sowohl bei erkrankten Tieren als auch bei Patienten mit Morbus Crohn in den entzündeten Darmabschnitten eine deutlich gesteigerte T-Zell-Reaktivität. Diese ist gekennzeichnet durch die verstärkte Expression von IL-12 und IFN-γ in den Läsionen. Demgegenüber ist das immunsupprimierende Zytokin IL-10 in den Läsionen deutlich erniedrigt (Immunity 3: 171-174, 1995; J. Exp. Med. 182:1281-1290, 1995; Eur. J. Immunol. 26:1156-1163; Eur. J. Immunol. 28: 379-389, 1998). Die Bedeutung des immunsupprimierenden Zytokins IL-10 für die Entwicklung entzündlicher Darmerkrankungen zeigt sich auch daran, daß IL-10 knockout Mäuse eine spontane Colitis entwickeln (Immunity 3: 171-174, 1995). Die Aktivierung der IFN-γ produzierenden T-Zellen in der Lamina propria des Darmes beruht im wesentlichen auf der lokalen Bildung von IL-12. In dem Tiermodell einer Allergen-induzierten Colitis konnte gezeigt werden, daß eine bestehende schwere Colitis mit Antikörpern gegen IL-12 zu therapieren ist. Die Neutralisierung von IL-12 mit Antikörpern führte zu einer klinischen und histopathologischen Normalisierung der Befunde innerhalb weniger Tage. Bei T-Zellen aus der Lamina propria der anti-IL-12-behandelten Mäuse ließ sich keine IFN-γ Bildung mehr nachweisen (J. Exp. Med. 182: 1281-1290).

Erste Anwendungen von rekombinantem IL-10 am Menschen bestätigen die entzündungshemmenden Eigenschaften. Nach Gabe von IL-10 an gesunde Probanden ist die Bildung der entzündungsfördernden Zytokine TNF-α und IL-1 durch ex vivo mit LPS aktivierte Monozyten um 65 bis 95% reduziert (J. Immunol. 154: 5492-5499, 1995). Die Anwendung von IL-10 bei Patienten mit Steroidrefraktärem Mobus Crohn resultiert in einer Verbesserung der klinischen Symptome (Gastroenterology, 113:383-389). Kürzlich wurde auch über die subkutane Anwendung von IL-10 bei 3 Patienten mit Psoriasis berichtet. Es kam zu einer Besserung der Krankheitssymptomatik. Ferner war die Bildung von IL-12 und TNF sowie die Expression von Oberflächenmolekülen auf Monozyten vermindert (J. Clin. Invest. 101:783-794). Die Anwendung von Antikörpern gegen IL-12 am Menschen steht derzeit bevor.
Zusammenfassend läßt sich feststellen, daß ein Mangel an IL-10 bzw. ein Überschuß an IL-12 die Pathophysiologie einer Vielzahl entzündlicher Erkrankungen bedingt. Ansätze zur Normalisierung der IL-10/IL-12 Balance haben daher ein großes therapeutisches Potential.

Eine Substanz mit dem immunmodulatorischen Wirkprinzip der IL-12 Hemmung und IL-10 Steigerung ist Thalidomid (J. Immunol. 159(10),5157-5161(1997)). Jedoch löst Thalidomid auch eine Reihe von Nebenwirkungen aus, zu denen Sedation, Teratogenität und Neuropathie zählen.

Die der Erfindung zugrundeliegende Aufgabe bestand somit in der Entwicklung von neuen Immunmodulatoren, die nicht zu einer generellen Immunsuppression führen und dabei eine Normalisierung der IL-10/IL-12 Balance bewirken.

Es wurde nun gefunden, daß die an die zu entwickelnden Substanzen gestellten Anforderungen von bestimmten substituierten Benzamiden erfüllt werden.
Gegenstand der Erfindung sind dementsprechend substituierte Benzamide der Formel I 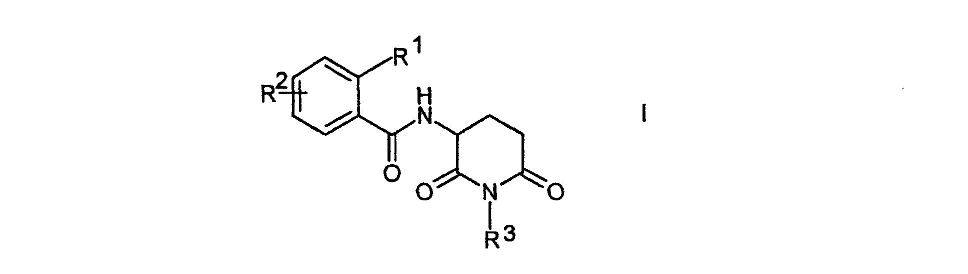 in der
- R¹: für eine Gruppe der Formel COOR⁴, in der R⁴ einen geradkettigen oder verzweigten Alkylrest mit 1-6 C-Atomen darstellt, oder für eine Gruppe der Formel CONR⁵R⁶, in der R⁵ und R⁶ gleich oder verschieden sind und eine Alkylgruppe mit 1-6 C-Atomen (geradkettig oder verzweigt) oder zusammen mit dem N-Atom einen Pyrrolidin-, Piperidin-, Hexamethylenimin- oder Morpholinring bedeuten, steht,
- R²: Chlor, Fluor, CF₃, einen Alkylrest mit 1-3 C-Atomen oder Wasserstoff bedeutet und
- R³: die Hydroxygruppe, einen Alkyl- oder Alkoxyrest mit 1-6 C-Atomen (geradkettig oder verzweigt und gegebenenfalls substituiert mit OH-, einer Alkoxy-, Ester- oder einer offenkettigen oder cyclischen Amidgruppe mit 1-6 C-Atomen) oder einen Rest CH₂-NR⁵R⁶, in dem R⁵ und R⁶ wie oben definiert sind, darstellt.

Die erfindungsgemäßen Verbindungen können sowohl in racemischer als auch enantiomerenreiner Form oder in Form von Salzen mit pharmazeutisch verträglichen Säuren vorliegen.

Bevorzugt werden substituierte Benzamide, in denen der Rest R¹ für eine Gruppe der Formel COOR⁴, in der R⁴ einen geradkettigen oder verzweigten Alkylrest mit 1-6 C-Atomen darstellt, oder für eine Gruppe der Formel CONR⁵R⁶, in der R⁵ und R⁶ zusammen mit dem N-Atom einen Morpholinring bedeuten, steht, R² H ist und R³ die Hydroxygruppe oder einen Rest CH₂-NR⁵R⁶, in dem R⁵ und R⁶ zusammen mit dem N-Atom einen Morpholinring bedeuten, darstellt.

Insbesondere bevorzugt sind das 2-(Morpholin-4-carbonyl)-N-(1-morpholin-4-ylmethyl-2,6-dioxo-piperidin-3-yl)-benzamid und der N-(1-Hydroxy-2,6-dioxo-piperidin-3-yl)-phthalamidsäure-methylester.

Verbindungen der allgemeinen Formel 1 lassen sich erhalten, indem man eine Carbonsäure der Formel II a oder II b 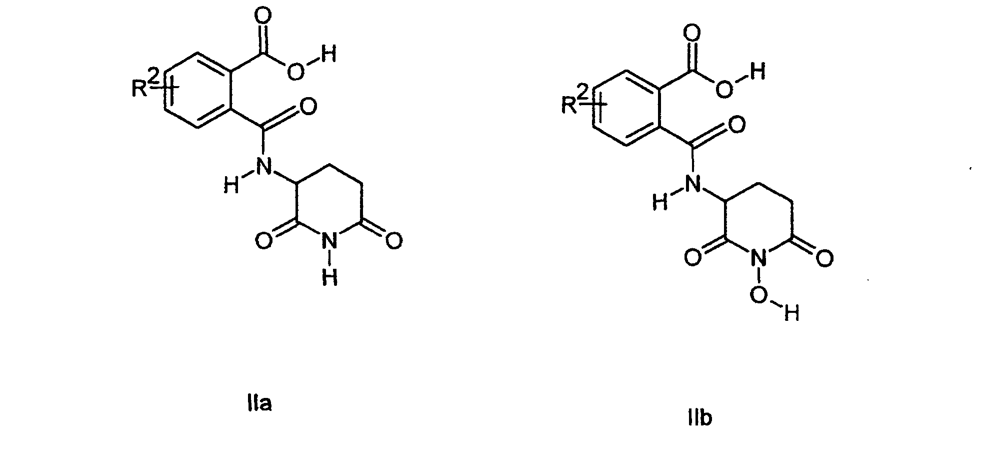 zunächst in an sich bekannter Weise in einen Ester (R¹ = COOR⁴) oder ein Amid (R¹ = CONR⁵R⁶) überführt. Dabei werden ausgehend von der Carbonsäure II b bereits erfindungsgemäße Verbindungen erhalten. In die entsprechend von der Carbonsäure II a abgeleiteten diese Verbindungen läßt sich dann in ebenfalls bekannter Weise, z.B. durch Mannich-Reaktion mit Paraformaldehyd und einem sekundären Amin der Formel HNR⁵R^{6,} der Rest R³ einführen, welcher von der Hydroxygruppe verschieden ist.

Weiterer Erfindungsgegenstand sind substituierte Benzamide der Formel I zur Verwendung als Wirkstoff in Arzneimitteln, insbesondere in Immunmodulatoren. Die erfindungsgemäßen Substanzen inhibieren die Bildung des entzündungsfördernden Zytokins IL-12 durch LPS-aktivierte humane Monozyten deutlich. Andererseits steigern Substanzen dieser Gruppe die Bildung des entzündungshemmenden Zytokins IL-10 durch LPS-aktivierte humane Monozyten. Dies unterscheidet die neuen Substanzen von bekannten Immunmodulatoren wie Steroiden und Phosphodiesterase-Inhibitoren, die sowohl die Synthese von IL-12 als auch die von IL-10 supprimieren. Aufgrund ihrer charakteristischen immunmodulatorischen Wirkung (Hemmung von IL-12, Steigerung von IL-10) sind die erfindungsgemäßen Substanzen für die Behandlung und/oder Prophylaxe von Entzündungen, insbesondere Entzündungen der Haut und Schleimhäute, der Gefäße sowie für die Behandlung und/oder Prophylaxe von Autoimmunerkrankungen geeignet.

Zu diesen Erkrankungen zählen unter anderem Entzündungen der Haut (z.B. atopische Dermatitis, Psoriasis, Ekzeme), Entzündungen der Atemwege (z.B. Bronchitis, Pneumonie, Asthma bronchiale, ARDS (adult respiratory distress syndrome), Sarkoidose, Silikose/Fibrose), Entzündungen des Gastrointestinaltraktes (z.B. gastroduodenale Ulcera, Morbus Crohn, ulcerative Colitis), ferner Erkrankungen wie Hepatitis, Pankreatitis, Appendizitis, Peritonitis, Nephritis, Aphthosis, Konjunktivitis, Keratitis, Uveitis, Rhinitis.
Die Autoimmunerkrankungen umfassen z.B. Erkrankungen des arthritischen Formenkreises (z.B. rheumatoide Arthritis, HLA-B27 assoziierte Erkrankungen), ferner Multiple Sklerose, jugendlicher Diabetes oder Lupus erythematodes.
Weitere Indikationen sind Sepsis, bakterielle Meningitis, Kachexie, Transplantat-Abstoßungsreaktionen, Graft-versus-Host Reaktionen sowie das Reperfusionssyndrom und Atherosklerose.

Erfindungsgemäße Arzneimittel enthalten neben mindestens einer Verbindung der allgemeinen Formel I Trägermaterialien, Füllstoffe, Lösungsmittel, Verdünnungsmittel, Farbstoffe und/oder Bindemittel. Die Auswahl der Hilfsstoffe sowie die einzusetzenden Mengen hängen davon ab, ob das Arzneimittel oral, intravenös, intraperitoneal, intradermal, intramuskulär, intranasal, buccal oder lokal appliziert werden soll. Für die orale Applikation eignen sich Zubereitungen in Form von Tabletten, Kautabletten, Dragees, Kapseln, Granulaten, Tropfen, Säften oder Sirupen, für die parentale, topische und inhalative Applikation Lösungen, Suspensionen, leicht rekonstituierbare Trockenzubereitungen sowie Sprays. Erfindungsgemäße Verbindungen in einem Depot in gelöster Form, einer Trägerfolie oder einem Pflaster, gegebenenfalls unter Zusatz von die Hautpenetration fördernden Mitteln, sind Beispiele für geeignete perkutane Applikationsformen. Aus oral oder perkutan anwendbaren Zubereitungsformen können die erfindungsgemäßen Verbindungen verzögert freigesetzt werden.

Die an Patienten zu verabreichende Wirkstoffmenge variiert in Abhängigkeit vom Gewicht des Patienten, von der Applikationsart, der Indikation und dem Schweregrad der Erkrankung. Üblicherweise werden 1 bis 150 mg/kg wenigstens einer erfindungsgemäßen Verbindung der Formel I appliziert.

### Beispiele

**Tabelle 1**

| | | |
|---|---|---|
| 4 (erfindungsgemäß) | R ¹ = COOCH ₃ | N-(1-Hydroxy-2,6-dioxo-piperidin-3-yl)-phthalamid-säure-methylester |
| | R ² = H | |
| | R ³ = OH | |
| 5 (erfindungsgemäß) | R ¹ = CONR ⁵ R ⁶ , | 2-(Morpholin-4-carbonyl)-N-(1-piperidin-4-ylmethyl-2,6-dioxo-piperidin-3-yl)benzamid |
| | wobei R ⁵ und R ⁶ zusammen mit dem N-Atom einen Morpholinring bedeuten | |
| | R ² = H | |
| | R ³ = CH ₂ -NR ⁵ R ⁶ , | |
| | wobei R ⁵ und R ⁶ zusammen mit dem N-Atom einen Piperidinring bedeuten | |
| 6 (erfindungsgemäß) | R ¹ = COOC ₂ H ₅ | N-(2,6-Dioxo-1-piperidin-1-yl-methyl-piperidin-3-yl)-phtalamid-säureethylester |
| | R ² = H | |
| | R ³ =CH ₂ -NR ⁵ R ⁶ , | |
| | wobei R ⁵ und R ⁶ zusammen mit dem N-Atom einen Piperidinring bedeuten | |

Die Substanzen der Tabelle 1 wurden ¹H-NMR-spektroskopisch charakterisiert (Gerät: DPX 300 Avance der Fa. Bruker; 300 MHz; Lösungsmittel: DMSO-d₆; Angabe der chemischen Verschiebung in ppm).

### Beispiel 1

1,97 - 2,16 (m, 2H, CH₂); 2,52 - 2,86 (m, 2H, CH₂); 3,20 - 3,75 (m, 8H, CH₂); 4,50 - 4,65 (m, 2H, NCH₂N); 4,64 - 4,82 (m, 1H, CH); 7,49 - 7,66 (m, 3H, aromat); 7,79 - 7,85 (d, 1H, aromat); 8,60 -8,72 (d, 1H, CONH).

### Beispiel 2

1,94 - 2,20 (m, 2H,CH₂); 2,68 - 2,95 (m, 2H, CH₂); 3,28 - 3,70 (m, 8H, CH₂); 4,52 - 4,65 (m, 2H, NCH₂N); 4,83 - 4,96 (m, 1H, CH); 7,42 - 7,58 (m, 3H, aromat); 7,85 - 7,92 (m, 2H, aromat) ; 8,85 - 8,89 (d, 1H, CONH).

### Beispiel 3

1,92 - 2,24 (m, 2H, CH₂); 2,69 - 3,02 (m, 2H, CH₂); 3,12 (s, 2H, CH₂); 3,35 - 3,77 (m, 12H, CH₂); 4,52 - 4,68 (m, 2H, NCH₂N); 4,74 - 4,98 (m, 1H, CH); 7,28 - 7,32 (d, 1H, aromat); 7,45 - 7,59 (m, 2H, aromat); 7,74 - 7,80 (d, 1H, aromat); 8,78 - 8,88 (d, 1H, CONH).

### Beispiel 4

2,01 - 2,18 (m, 2H, CH₂); 2,58 - 2,88 (m, 2H, CH₂); 3,79 (s, 3H, COOCH₃); 4,70 - 4,80 (m, 1H, CH); 7,54 - 7,72 (m, 3H, aromat); 7,77 - 7,81 (d, 1H, aromat); 8,77 - 8,82 (d, 1H, CONH); 10,21 (s, 1H, NOH).

### Beispiel 5

1,38 - 1,46 (m, 6H, CH₂); 1,96 - 2,16 (m, 2H, CH₂); 2,20 - 2,32 (m, 4H, CH₂); 2,60 - 2,92 (m, 2H, CH₂); 3,26 - 3,75 (m, 8H, CH₂); 4,57 - 4,70 (m, 2H, NCH₂N); 4,72 - 4,88 (m, 1H, CH); 7,28 - 7,30 (d, 1H, aromat); 7,43 - 7,57 (m, 2H, aromat); 7,70 - 7,74 (m, 1H, aromat); 8,75 - 8,78 (d, 1H, CONH).

### Beispiel 6

1,18 - 1,26 (t, 3H, CH₃); 1,36 - 1,48 (m, 6H, CH₂); 1,95 - 2,16 (m, 2H, CH₂); 2,22 - 2,34 (m, 4H, CH₂); 2,60 - 2,90 (m, 2H, CH₂); 4,08 - 4,18 (q, 2H, OCH₂); 4,55 - 4,68 (m, 2H, NCH₂N); 4,70 - 4,80 (m, 1H, CH); 7,54 - 7,72 (m, 3H, aromat); 7,78 - 7,83 (d, 1H, aromat); 8,78 - 8,82 (d, 1H, CONH).

### Untersuchung der immunmodulatorischen Wirksamkeit

Humane Monozyten wurden aus peripheren Blut-mononucleären Zellen (PBMC), die mittels einer Ficoll-Dichtegradientenzentrifugation von heparinisiertem Vollblut gewonnen wurden, isoliert. Dazu wurden die PBMC mit einem monoklonalen Antikörper inkubiert, der gegen das Monozyten-spezifische Oberflächenmolekül CD14 gerichtet ist und an den superparamagnetische Microbeads (Miltenyi Biotech, Bergisch Gladbach) gekoppelt sind. Zur positiven Selektion der markierten Monozyten aus dem Zellgemisch der PBMC wurde die Gesamtzellsuspension auf eine Säule mit ferromagnetischer Trägermatrix aufgebracht und diese in ein Magnetfeld gestellt. Dadurch wurden die Zellen, die mit Microbeads beladen waren, an die Trägermatrix gebunden, unmarkierte Zellen passierten die Säule und wurden verworfen. Nach Herausnehmen der Matrix aus dem Magnetfeld wurden die Antikörperbeladenen Zellen durch Spülen der nun entmagnetisierten Säule mit Puffer eluiert. Die Reinheit dieser so erhaltenen CD14-positiven Monozytenpopulation betrug etwa 95 bis 98%. Diese Monozyten wurden in einer Dichte von 10⁶ Zellen/ml Kulturmedium (RPMI, supplementiert mit 10% foetalem Kälberserum) mit den in DMSO-gelösten Prüfsubstanzen für eine Stunde bei 37°C und 5% CO₂ inkubiert. Anschließend wurde 20 µg/ml LPS aus E. coli zugegeben. Nach 24 Stunden wurden zellfreie Kulturüberstände genommen und auf den Gehalt an den Zytokinen IL-12 und IL-10 getestet.

Die Konzentration von IL-12 und IL-10 in den Zellkulturüberständen wurde mittels Sandwich-ELISAs unter Verwendung zweier anti-IL-12 bzw. anti-IL-10 monoklonaler Antikörper (Biosource Europe, Fleurus, Belgien) bestimmt. Eine Referenzstandardkurve mit humanem IL-12 bzw. IL-10 wurde eingeschlossen. Das Detektionslimit des IL-12 ELISAs betrug 10 pg/ml, das des IL-10 ELISAs 15 pg/ml.

**Tabelle 2**

| Einfluß der Prüfsubstanzen auf die IL-12 und IL-10 Produktion von LPS-aktivierten Monozyten. | | | |
|---|---|---|---|
| Substanz | Konzentration | IL-12 Produktion % der Konrolle (= 100%) | IL-10 Produktion % der Kontrolle (= 100%) |
| aus Beispiel 1 | 10 µg/ml | 76 | 109 |
| | 2 µg/ml | 78 | 84 |
| | 0.4 µg/ml | 90 | 87 |
| aus Beispiel 2 | 6.0 µg/ml | 105 | 69 |
| | 2.0 µg/ml | 104 | 99 |
| | 0.66 µg/ml | 102 | 109 |
| | 0.22 µg/ml | 99 | 114 |
| aus Beispiel 3 | 10 µg/ml | 30 | 127 |
| | 2 µg/ml | 46 | 121 |
| | 0.4 µg/ml | 87 | 108 |
| aus Beispiel 3 (zweiter Versuch) | 6.0 µg/ml | 49 | 131 |
| | 2.0 µg/ml | 53 | 133 |
| | 0.66 µg/ml | 59 | 123 |
| | 0.22 µg/ml | 64 | 117 |
| aus Beispiel 4 | 10 µg/ml | 23 | 112 |
| | 2 µg/ml | 40 | 189 |
| | 0.4 µg/ml | 66 | 122 |
| aus Beispiel 5 | 6.0 µg/ml | 47 | 134 |
| | 2.0 µg/ml | 57 | 134 |
| | 0.66 µg/ml | 67 | 122 |
| | 0.22 µg/ml | 88 | 115 |
| aus Beispiel 6 | 6.0 µg/ml | 38 | 130 |
| | 2.0 µg/ml | 47 | 124 |
| | 0.66 µg/ml | 61 | 132 |
| | 0.22 µg/ml | 79 | 102 |
| Dexamethason | 1 µM | 6 | 34 |
| | 0.1 µM | 6 | 35 |
| | 0.01 µM | 20 | 64 |
| | 0.001 µM | 83 | 105 |
| Pentoxifyllin | 50 µg/ml | 74 | 74 |
| | 5 µg/ml | 72 | 81 |
| Rolipram | 50 µM | 32 | 28 |
| | 0.5 µM | 30 | 79 |
| | 0.005 µM | 58 | 92 |

Die in Tabelle 2 dargestellten Ergebnisse zeigen, daß bekannte Immunmodulatoren wie Dexamethason, Pentoxifyllin und Rolipram bei LPS-aktivierten Monozyten sowohl die Bildung von IL-12 als auch die von IL-10 unterdrücken. Im Vergleich zeigen strukturnahe mit Carboxygruppen substituierte Benzamide (Beispiel 1) bei hohen Dosierungen eine nur geringfügige Wirkung.

Überraschenderweise sind die erfindungsgemäßen Ester (Beispiele 4 und 6) und die erfindungsgemäßen Amide (Beispiele 3 und 5) der substituierten Benzamide in dem untersuchten Modell immunmodulatorisch wirksam. Derartige Verbindungen hemmen die Synthese von IL-12 durch LPS-aktivierte Monozyten bei Konzentrationen von 10, 6 bzw. 2 µg/ml in potenter Weise. Im Gegensatz zu den bekannten Immunmodulatoren steigern sie aber auch die Synthese von IL-10. Dieses charakteristische Muster (deutliche Hemmung von IL-12, Steigerung von IL-10 in demselben Konzentrationsbereich der Substanzen) kennzeichnet einen neuartigen Typ von Immunmodulator.

## Patentansprüche

1. Substituierte Benzamide der Formel I 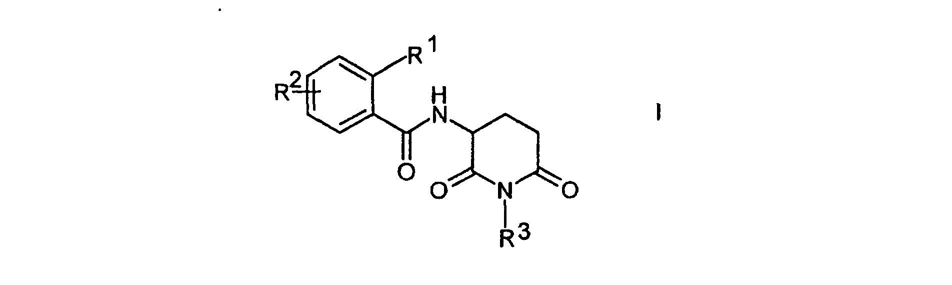 in der
R¹ für eine Gruppe der Formel COOR⁴, in der R⁴ einen geradkettigen oder verzweigten Alkylrest mit 1-6 C-Atomen darstellt, oder für eine Gruppe der Formel CONR⁵R⁶, in der R⁵ und R⁶ gleich oder verschieden sind und eine Alkylgruppe mit 1-6 C-Atomen (geradkettig oder verzweigt) oder zusammen mit dem N-Atom einen Pyrrolidin-, Piperidin-, Hexamethylenimin- oder Morpholinring bedeuten, steht,
R² Chlor, Fluor, CF₃, einen Alkylrest mit 1-3 C-Atomen oder Wasserstoff bedeutet und
R³ die Hydroxygruppe, einen Alkyl- oder Alkoxyrest mit 1-6 C-Atomen (geradkettig oder verzweigt und gegebenenfalls substituiert mit OH-, einer Alkoxy-, Ester- oder einer offenkettigen oder cyclischen Amidgruppe mit 1-6 C-Atomen) oder einen Rest CH₂-NR⁵R⁶, in dem R⁵ und R⁶ wie oben definiert sind, darstellt.

2. Substituiertes Benzamid der Formel I gemäß Anspruch 1, **dadurch gekennzeichnet, daß**
R¹ für eine Gruppe der Formel COOR⁴, in der R⁴ einen geradkettigen oder verzweigten Alkylrest mit 1-6 C-Atomen darstellt, oder für eine Gruppe der Formel CONR⁵R⁶, in der R⁵ und R⁶ zusammen mit dem N-Atom einen Morpholinring bedeuten, steht,
R² H ist und
R³ die Hydroxygruppe oder einen Rest CH₂-NR⁵R⁶, in dem R⁵ und R⁶ zusammen mit dem N-Atom einen Morpholinring bedeuten, darstellt.

3. Substituierte Benzamide der Formel 1 gemäß Anspruch 1 oder 2 zur Verwendung als Wirkstoff in einem Arzneimittel.

4. Substituierte Benzamide der Formel 1 gemäß Anspruch 1 oder 2 zur Verwendung nach Anspruch 3, **dadurch gekennzeichnet, daß** das Arzneimittel ein Immunmodulator ist.

## Claims

1. Substituted benzamides corresponding to formula I 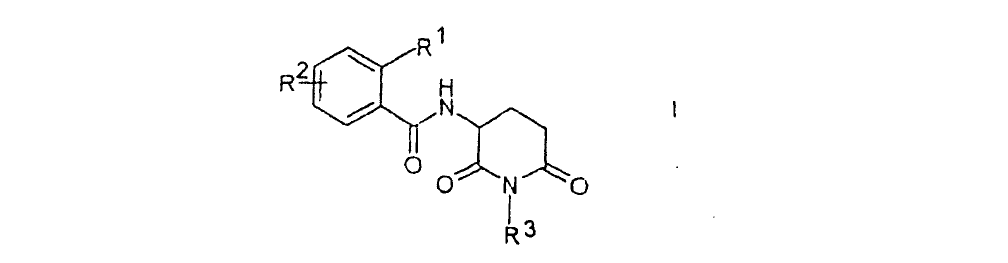 wherein
R¹ represents a group corresponding to the formula COOR⁴, wherein R⁴ denotes a straight-chain or branched alkyl group having 1 to 6 C atoms, or a group corresponding to the formula CONR⁵R⁶, wherein R⁵ and R⁶ are identical or different and denote an alkyl group having 1 to 6 C atoms (straight-chain or branched) or, together with the N atom, denote a pyrrolidine, piperidine, hexamethyleneimine or morpholine ring,
R² denotes chlorine, fluorine, CF₃, an alkyl group having 1 to 3 C atoms or hydrogen and
R³ denotes the hydroxyl group, an alkyl or alkoxy group having 1 to 6 C atoms (straight-chain or branched and optionally substituted with OH-, an alkoxy group, ester group or an open-chain or cyclic amide group having 1 to 6 C atoms) or a group CH₂-NR⁵R , wherein R⁵ and R⁶ are as defined above.

2. Substituted benzamide corresponding to formula I according to claim 1, **characterised in that**
R¹ represents a group corresponding to the formula COOR⁴, wherein R⁴ denotes a straight-chain or branched alkyl group having 1 to 6 C atoms, or a group corresponding to the formula CONR⁵R⁶, wherein R⁵ and R⁶ together with the N atom denote a morpholine ring;
R² is H and
R³ denotes the hydroxyl group or a group CH₂-NR⁵R⁶, wherein R⁵ and R⁶ together with the N atom denote a morpholine ring.

3. Substituted benzamides corresponding to formula I according to claim 1 or 2 for use as active ingredient in a medicament.

4. Substituted benzamides of the formula I according to claim 1 or 2 for the use according to claim 3, **characterised in that** the medicament is an immunomodulator.

## Revendications

1. Benzamides substitués de formule I 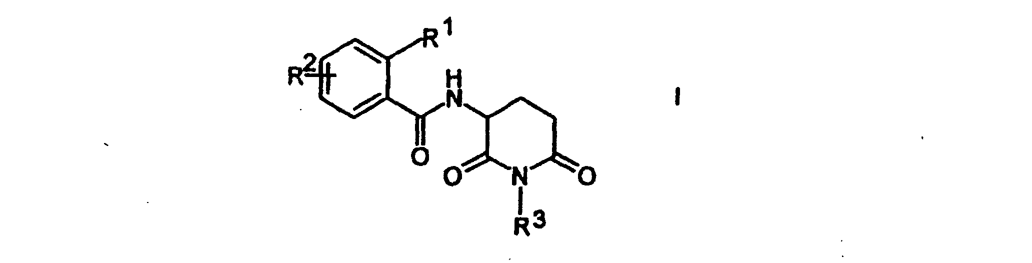 dans laquelle
R¹ est un groupe de formule COOR⁴, dans laquelle R⁴ représente un reste alkyle linéaire ou ramifié ayant 1 à 6 atomes de carbone,.ou un groupe de formule CONR⁵R⁶, dans laquelle R⁵ et R⁶ sont identiques ou différents et représentent un groupe alkyle (linéaire
ou ramifié) ayant 1 à 6 atomes de carbone ou forment conjointement avec l'atome d'azote un noyau de pyrrolidine, pipéridine, hexaméthylène-imine ou morpholine,
R² représente le chlore, le fluor, un reste CF₃, un reste alkyle ayant 1 à 3 atomes de carbone ou l'hydrogène et
R³ représente le groupe hydroxy, un reste alkyle ou alkoxy ayant 1 à 6 atomes de carbone (linéaire ou ramifié et substitué le cas échéant avec un groupe OH, un groupe alkoxy, un groupe ester ou un groupe amide à chaîne ouverte ou cyclique ayant 1 à 6 atomes de carbone) ou un reste CH₂-NR⁵R⁶ dans lequel R⁵ et R⁶ sont tels que définis ci-dessus.

2. Benzamide substitué de formule I suivant la revendication 1, **caractérisé en ce que**
R¹ représente un groupe de formule COOR⁴, dans laquelle R⁴ est un reste alkyle linéaire ou ramifié ayant 1 à 6 atomes de carbone, ou représente un groupe de formule CONR⁵R⁶, dans laquelle R⁵ et R⁶ forment un noyau de morpholine conjointement avec l'atome d'azote,
R² représente H et
R³ est un groupe hydroxy ou un reste CH₂-NR⁵R⁶ dans lequel R⁵ et R⁶ forment un noyau morpholine conjointement avec l'atome d'azote.

3. Benzamides substitués de formule I suivant la revendication 1 ou 2, destinés à être utilisés comme substance active dans un médicament.

4. Benzamides substitués de formule I suivant la revendication 1 ou 2 destinés à être utilisés selon la revendication 3, **caractérisés en ce que** le médicament est un immunomodulateur.
